## Europäisches Patentamt
## European Patent Office
## Office européen des brevets

(11) Veröffentlichungsnummer: **0 286 679**
**A1**

(12) # EUROPÄISCHE PATENTANMELDUNG
veröffentlicht nach Art. 158 Abs. 3 EPÜ

(21) Anmeldenummer: 87900352.3

(22) Anmeldetag: 29.10.86

Daten der zugrundeliegenden internationalen Anmeldung:

(86) Internationale Anmeldenummer:
PCT/SU 86/00109

(87) Internationale Veröffentlichungsnummer:
WO 88/03009 (05.05.88 88/10)

(51) Int. Cl.⁴: **A 61 F 2/16**

(43) Veröffentlichungstag der Anmeldung: 19.10.88
Patentblatt 88/42

(84) Benannte Vertragsstaaten: **BE DE FR GB IT LU NL**

(72) Erfinder: FEDOROV, Svyatoslav Nikolaevich, ul.
Dostoevskogo, 12-32, Moscow, 103030 (SU)
Erfinder: PASHINOVA, Nadezhda Fedorovna, ul.
Koshtoyantsa, 32-60, Moscow, 118454 (SU)
Erfinder: ANISIMOV, Sergei Igorevich, ul.
Ostrovityanova, 26-3-13, Moscow, 117321 (SU)
Erfinder: DEGTEV, Evgeny Ivanovich, Yaroslavskoe
shosse, 14-34, Moscow, 129348 (SU)
Erfinder: ZAKHAROV, Dmitry Valerievich, ul.
Dubninskaya, 71-109, Moscow, 127591 (SU)
Erfinder: KARAVAEV, Alexandr Alexandrovich,
Michurinsky pr., 22/3-30, Moscow, 117191 (SU)
Erfinder: KISELEV, Vladimir Grigorievich, ul.
Musorgskogo, 1-30, Moscow, 127490 (SU)
Erfinder: JUZHELEVSKY, July Abramovich, ul.
Budapeshtskaya, 38-2-227, Leningrad, 192071 (SU)
Erfinder: SOKOLOV, Sergei Vasilievich, pr.
Syslova, 19-1-222, Leningrad, 198217 (SU)

(71) Anmelder: MOSKOVSKY
NAUCHNO-ISSLEDOVATELSKY INSTITUT
MIKROKHIRURGII GLAZA, Beskudnikovsky bulvar, 59a,
Moscow, 127486 (SU)

(74) Vertreter: Finck, Dieter et al, Patentanwälte v. Füner,
Ebbinghaus, Finck Mariahilfplatz 2 & 3,
D-8000 München 90 (DE)

(54) **KRISTALLINE KÜNSTLICHE HINTERKAMMERLINSE.**

(57) In Hinterkammer einsetzbare, künstliche Augenlinse, die eine Linse (1) selbst und zwei daran diametral befestigte Stützelemente (2) vorsieht, wobei jedes Stützelement (2) einen Körper der krummlinigen Gestalt darstellt, bei dem eine der Seitenflächen die konvexe ist und mit ihrer Konvexität (3) von der Hauptebene der Linse (1) abgewandt ist, und die andere Seitenfläche des erwähnten Körpers die konkave ist und mit ihrer Konkavität (4) der Hauptebene der Linse (1) zugekehrt ist, während die Enden der betreffenden Stützelemente (2) abgerundet ausgeführt ein dem anderen zugewandt sind, wobei die genannten Stützelemente (2) in bezug auf die Hauptebene in entgegengesetzten Richtungen ein gegen das andere räumlich verdreht sind.

EP 0 286 679 A1

# IN HINTERKAMMER EINSETZBARE KÜNSTLICHE AUGENLINSE

## Technisches Gebiet

Die vorliegende Erfindung betrifft Prothesen und Mittel zu deren Festlegung im Körper des Menschen, insbesondere in Hinterkammer einsetzbare, künstliche Augenlinsen.

## Zugrundeliegender Stand der Technik

Wie bekannt tritt eine Notwendigkeit bei dem chirurgischen Behandlung verschiedenartiger Erkrankungen des Auges des öfteren auf, die natürliche Augenlinse zu entfernen und anschließend durch eine künstliche Augenlinse, Intraokularlinse dieselbe zu ersetzen. In der Regel verläuft die Implantation der künstlichen Augenlinsen in die Hinterkammer des Auges unter Schwierigkeiten wegen der auftretenden Komplikationen in Form von Rissen der hinteren Linsenkapsel und des Herausfalls des Glaskörpers.

Bekannt ist eine künstliche Augenlinse von Shiering (The Journal of the American Academy of the Ophthalmology, 1982, 89, Nr. 8 S, S. 128), die eine Linse selbst und zwei diametral gegenüberliegende Abstützungselemente vorsieht, welche in einer mit der Hauptebene der Linse zusammenfallenden Ebene liegen. Die Implantation dieser künstlichen Augenlinse ist komliziert, da bei der Einführung der erwähnten Abstützungselemente in die spaltförmige Hinterkammer in dieser Faltenbildung der hinteren Kapsel des öfteren mit sich bringt, die im Laufe der weiteren Implantation zum Zerreißen der hinteren Linsenkapsel führen, wodurch die Irrigation des Auges durchgeführt werden soll. In dieser Hinsicht wird die Zeitdauer der Operation verlängert, deren Bequemlichkeit herabgesetzt, die Anzahl der Ödeme an der Hornhaut vergrößert, die durch Einwirkung am Auge der Irrigationsflüssigkeit entstehen. In diesem Zusammenhang werden die Friste der Nachoperationsbehandlung der Patienten verlängert. Darüber hinaus stellt die erwähnte Anordnung der Abstützungselemente kein Hindernis an der Versetzung der künstlichen Augenlinse während der Nachoperationsperiode dar.

Im weteren ist noch eine künstliche Augenlinse vom Pirs bekannt (The Journal of the American Academy of the Ophthalmology, 1982, 89, Nr. 8 S, S. 30), die die Linse selbst und drei radialverlaufende Stützelemente vorsieht, welche in der Hauptebene der Linse liegen. Diese künstliche Augenlinse wird auch in die Hinterkammer des Auges eingeführt. Zu diesem Zweck wird diese zunächst nur mit einem Stützelement in die Hinterkammer des Auges hineingeschoben, während die anderen zwei Stützelemente durch Beiseiteschieben und Darauflegen der Regenbogenhaut auf diese zurechtgesetzt werden. Dabei wird die Implantation wieder des öfteren mit dem Zerreißen der Hinterkapsel der Augenlinse begleitet, da das flache Stützelement sich bei dessen Implantation in die Hinterkammer mit seinem Rand in Richtung zu der hinteren Linsenkapsel unter einem gewissen Winkel verstellt. Dabei stößt der Rand des erwähnten Stützelementes gegen die Kapsel und kann bei seiner weiteren Verstellung diese verletzen. Darüber hinaus muß die Tatsache durch die erwähnte Anordnung der Stützelemente in der Hauptebene der Linse in Kauf genommen werden, daß die Linse sich gegen die Sehachse des öfteren versetzt, indem dadurch das Sehvermögen verschlechtert wird. Zu deren zuverlässiger Festlegung ist eine die Linse an der Regenbogenhaut befestigende Zusatznaht anzulegen. Dadurch wird die Operation verlängert und deren Fähigkeit zu verletzen vergrößert, wodurch letzten Endes die Fristen der Nachoperationsbehandlung der Kranken verlängert werden.

### Offenbarung der Erfindung

Der Erfindung liegt die Aufgabe zugrunde, eine in Hinterkammer einsetzbare, künstliche Augenlinse zu entwickeln, deren Stützelemente in ihrer Gestaltung die Entstehung der Operationskomplikationen verhindern.

Die gestellte Aufgabe wird dadurch gelöst, das in der in der Hinterkammer einsetzbaren, künstlichen Augenlinse, die die Linse selbst und mindestens zwei diametral befestigte Stützelemente vorsieht, erfindungsgemäß jedes Stützelement einen Körper der krummlinigen Gestalt darstellt, wobei eine dessen Seitenflächen eine konvexe ist und mit ihrer Konvexität von der Hauptebene der Linse abgewandt ist, während die andere Körperseite die Konkavfläche aufweist, die mit ihrer Konkavität der Hauptebene der Linse zugekehrt ist, und die Enden der Stützelemente abgerundet ausgeführt und ein dem anderen entgegen verlegt sind, wobei noch die Stützelemente im Raum

00286679

in bezug auf die Hauptebene der Linse ein gegen anderes in entgegengesetzten Richtungen gewendet sind.

Durch die erfindungsgemäße Ausführung der in Hinterkammer einsetzbaren künstlichen Augenlinse wird eine ganze Reihe von wesentlichen Vorteilen erreicht, die nachstehend kurz dargelegt werden.

Durch Benutzung in der erfindungsgemäßen künstlichen Augenlinse mindestens zwei Stützelemente, die einen Körper der krummlinigen Gestalt darstellen, dessen eine der beiden Seiten die konvexe Fläche darstellt, ermöglicht die Augenlinse in der Hinterkammer bzw. in der Linsenkapsel festzulegen. Dabei stellen die von der Hauptebene der Linse abgewandten Konvexitäten einen Teil der künstlichen Augenlinse dar, der mit den Geweben des Auges in Berührung tritt.

Durch das Vorliegen der Konkavitäten an den Stützelementen wird die Möglichkeit mit der in Hinterkammer einsetzbaren künstlichen Augenlinse die Regenbogenhaut zu verletzen auf das kleinste reduziert, was auf die Verminderung der Berührungsfläche zwischen der Regenbogenhaut und den Stutzelementen zurückzuführen ist, da das in das Auge implantierte Stützelement nach eine Verdrehung "auf die Rippe" strebt. Gerade zu diesem Augenblick ermöglicht die vorhandene Konkavität den verletztenden Eingriff der Augenlinse zu vermeiden. Die räumliche Verdrehung der Stützelemente bezüglich der durch den optischen Mittelpunkt der Linse verlegten Hauptebene in ein gegen das andere entgegengesetzten Richtungen ermöglicht es, den spaltförmigen Raum der Hinterkammer bei der Implantation durch Verdrehung der Linse in die Waagerechtebene zu erweitern. In diesem Zusammenhang besteht die Möglichkeit, das Stützelement ohne eine zusätzliche Irrigation in die Hinterkammer ungehindert einzuführen. Durch die erwähnte Erweiterung zwischen der Hinterfläche der Regenbogenhaut und der hinteren Linsenkapsel mit dem im Raum verdrehten Stützelement bei der Implantation wird die Anzahl der Risse der hinteren Linsenkapsel mit dem Herausfall des Glaskörpers bei dieser Operationsphase herabgesetzt, die Implantation vereinfacht und die Zeitdauer der Operation verkürzt. Darüber hinaus bringt die erwähnte Verdrehung der Stützelemente im Raum die Abstützung der

00286679

künstlichen Linse durch die Seitenränder der Einstellungsstützelemente an der Linsenkapsel mit sich, wodurch die Zuverlässigkeit der Linsenfestlegung gesteigert und somit die Möglichkeit der Dezentrierung der Linse herabgesetzt wird. Die entgegenstreckende Ausrichtung der Stützelemente ein zu dem anderen und deren Abrundung sind aus dem Grunde anzubringen, damit die abgewendeten Stützelemente die Endothelschicht der Regenbogenhaut im Laufe der Implantation keinesfalls verletzen und die Augenlinse an deren Verdrehung in der Hinterkammer bei dem Hineinschieben der Stützelemente in diese verhindern.

## Kurzbeschreibung der Zeichnungen

Nachstehend wird die Erfindung anhand eines Beispiels deren Ausführung unter Bezugnahme auf angelegte Zeichnungen näher erläutert, in denen es zeigt:

Fig. 1 erfindungsgemäße, in die Hinterkammer einsetzbare künstliche Augenlinse in Gesamtansicht;

Fig. 2 dieselbe in erfindungsgemäßer Seitenansicht;

Fig. 3 dieselbe in erfindungsgemäßer Draufsicht;

Fig. 4 bis 10 Phasen der Implantation der erfindungsgemäßen in die Hinterkammer einsetzbaren künstlichen Augenlinse in das Auge.

## Beste Ausführungsvariante der Erfindung

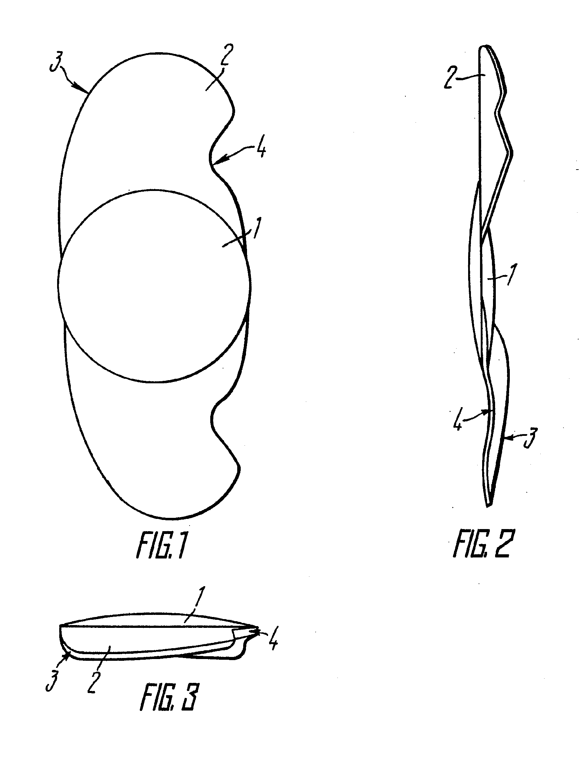

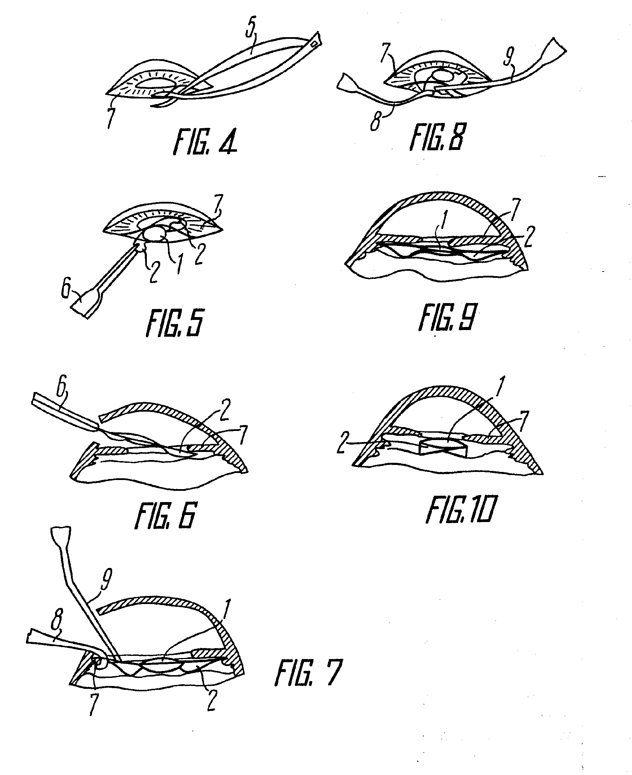

Die in der Hinterkammer einsetzbare, künstliche Augenlinse enthält eine Linse 1 (Fig. 1) selbst und zwei diametral befestigte Stützelemente 2, wobei jedes Stützelement 2 einen Körper der krummlinigen Gestalt darstellt. Eine der Seiten des erwähnten Körpers stellt die konvexe Fläche dar, die mit ihrer Konvexität 3 von der Hauptebene der Linse 1 abgewandt ist, während die andere Körperseite eine konkave Fläche ist und mit ihrer Konkavität 4 der Hauptebene der Linse 1 zugekehrt ist, wobei die Enden der genannten Stützelementen 2 abgerundet ausgeführt sind und ein zu dem anderen entgegenlaufen, dabei werden die Stützelemente 2 im Raum bezüglich der durch den optischen Mittelpunkt der Linse 1 hindurchgehenden Hauptebene ein gegen das andere in entgegengesetzten Richtungen gewendet, wie dies Fig. 2 und 3 wiedergeben.

Die bereits beschriebene, in der Hinterkammer einsetzbare künstliche Augenlinse kann durch Gießen aus einem besonderen elastischen Werkstoff hergestellt werden.

Die Implantation der erfindungsgemäßen, in der Hinterkammer einsetzbaren, künstlichen Augenlinse wird in der in den Fig. 4 bis 8 dargestellten Reihenfolge durchgeführt. Durch einen korneoskleralen Einschnitt vermittels der Schere 5 mit einer Länge von 3 mm längs des Meridians 17 Uhr unter örtlicher Betäubung wird in die Vorderkammer des Auges das Irrigationssystem eingeführt, wonach der Einschnitt 4 bis 6 mm an dem Limbus entlang zwischen 11 bis 14 Uhr (Fig. 4) verlegt wird. Die Vorderkapsel der Augenlinse wird in an sich bekannter Weise wie bei der extrakapsulären Extraktion der Katarakt eröffnet. Der Kern und die Linsenmassen werden ebenfalls typisch wie bei der extrakapsulären Extraktion der Katarakt entfernt. Hiernach wird die künstliche Augenlinse durch die Pinzette 6 (Fig. 5, 6) an einem der Stützelemente 2 ergriffen und mit dem anderen freien Stützelement 2 in die aus der Zeichnung nicht ersichtliche Hinterkammer des Auges eingeführt. Alsdann wird die Linse 1 selbst der künstlichen Augenlinse in der Waagerechtebene gedreht, mit der sich auch das im Sehlochbereich befindliche Stützelement 2 in bezug auf die Waagerechtebene verschwenkt und die Hinterseite der Regenbogenhaut 7 von der Linsenkapsel wegschiebt, wodurch die Möglichkeit entsteht, das erwähnte Stützelement 2 vollkommen in die Hinterkammer ungehindert einzuschieben. Hiernach wird der obere Teil der Regenbogenhaut 7 (Fig. 7) durch den Iridoretraktor 8 zurückgezogen, wonach das Stützelement 2 mit dem Spatel 9 (Fig.8) in die Hinterkammer hineingetaucht wird, wodurch die künstliche Augenlinse in der Hinterkammer des Auges ihre Waagerechtstellung (Fig. 9, 10) einnimmt.

Bei der Implantation der künstlichen Augenlinse wird eine kleinstmögliche Verletzbarkeit an der Regenbogenhaut 7 durch die krummlinige Gestaltung der Stützelemente 2 und durch das Vorliegen der Konkavität 4 infolge der Verminderung der Kontaktfläche zwischen der Regenbogenhaut 7 und den genannten Stützelementen 2 erreicht, da das als ersteres in die Hinterkammer implantierte Stützelement 2 sich parallel der Regenbogenhaut 7 zu verdrehen strebt und dadurch das noch in der Vorderkammer verbleibende andere Stützelement 2 an die Seitenfläche dreht.

- 6 -

Diese Verdrehung der Stützelemente im Raum bezüglich der Hauptebene in entgegengesetzten Richtungen gegeneinander ermöglicht es, die in die Hinterkammer einsetzbare künstliche Augenlinse in die erwähnte Hinterkammer ungehindert einzuführen. Dieser Effekt wird durch Auseinanderschieben der Hinterfläche der Regenbogenhaut und der Hinterkapsel der Linse durch die verdrehten Stützelemente erreicht, wodurch auch die Anzahl der Zerreissungen der Hinterkapsel der Linse mit dem Herausfallen des Glaskörpers bei der Implantation der künstlichen Augenlinse in die Hinterkammer des Auges herabgesetzt wird. Die Ausrichtung der Stützelemente ein dem anderen entgegen und deren Abrundung sind dazu notwendig, damit die räumlich verdrehten Stützelemente die Endothelschicht der Regenbogenhaut nicht verletzen und die Augenlinse in der Hinterkammer bei der Implantation an deren Verdrehung verhinderten.

Der Kranke M., 64 Jahre alt, ist in die Klinik mit DS:OD Altersstar eingeliefert worden. Die Sehschärfe bei der Einlieferung:

OD = richtige Lichtprojektion,

OS = 0,9 nicht korrigiert.

Es ist eine Operation durchgeführt worden: extrakapsulare Extraktion der Katarakt mit der Implantation der in der Hinterkammer einsetzbaren künstlichen Augenlinse + 24,0 im rechten Auge.

Die Operation selbst und die Nachoperationsperiode sind ohne Komplikationen vergangen. In drei Tagen nach der Operation ist er entlassen worden. Bei der Entlassung war die Sehschärfe:

OD = 1,0 ohne Korrektion,

OS = 0,9 nicht Korrigiert

Die Kranke V., 45 Jahre alt, ist in die Klinik mit DS:OD Cataracta complicata. Die Sehschärfe bei der Einlieferung:

OD = 1,0

OS = 0,01 nicht korrigiert.

Es ist eine extrakapsulare Extraktion der Katarakt mit der Implantation der in der Hinterkammer einsetzbaren, künstlichen Augenlinse in das linke Auge durchgeführt worden. Die Operation selbst und die Nachoperationsperiode sind ohne Komplikationen vergangen. In 5 Tagen nach der Operation entlassen. Die Sehschärfe bei der Entlassung:   OD = 1,0

OS = 1,0 ohne Korrektion.

Die erfindungsgemäse in der Hinterkammer einsetzbare, künstliche Augenlinse ist bei der intraokularen Korrektion der Aphakie nach der Extraktion der Katarakt beliebiger Ätiologie von Nutzen.

### Industrielle Anwendbarkeit

Die vorliegende Erfindung kann in der Ophthalmologie für die intraokulare Korrektion des Sehens nach der Extraktion von Katarakt beliebiger Ätiologie ihre Anwendung finden.

00285579

PATENTANSPRUCH:

In der Hinterkammer einsetzbare künstliche Augenlinse, die die Linse (1) selbst und mindestens zwei daran diametralbefestigten Stützelemente (2) vorsicht, d a d u r c h   g e - k e n n z e i c h n e t, daß jedes Stützelement (2) einen Körper der krummlinigen Gestalt darstellt, wobei eine der Seitenflächen des erwähnten Körpers die konvexe ist und mit ihrer Konvexität (3) von der Hauptebene der Linse (1) abgewandt ist, während die andere Seitenfläche des Körpers die konkave Fläche ist und mit ihrer Konkavität (4) der Hauptebene der Linse (1) zugekehrt ist, die Enden der betreffenden Stützelemente (2) abgerundet ausgeführt und ein dem anderen zugewandt sind, wobei die erwähnten Stützelemente (2) im Raum bezüglich der Hauptebene in entgegengesetzten Richtungen ein gegen das andere verdreht sind.

FIG. 1

FIG. 2

FIG. 3

FIG. 4

FIG. 8

FIG. 5

FIG. 9

FIG. 6

FIG. 10

FIG. 7

# INTERNATIONAL SEARCH REPORT

00285679

International Application No PCT/SU 86/00109

## I. CLASSIFICATION OF SUBJECT MATTER (if several classification symbols apply, indicate all) 6

According to International Patent Classification (IPC) or to both National Classification and IPC

IPC⁴    A61F 2/16

## II. FIELDS SEARCHED

| Minimum Documentation Searched 7 | |
|---|---|
| Classification System | Classification Symbols |
| IPC⁴ | A61F 1/16,2/16 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | US, A, 4159546 (Steven P. Shearing) 03 July 1979 (03.07.79) see the abstract | 1,2 |
| A | US, A, 4418431 (Fred T. Feaster) 06 December 1983 (06.12.83) see the abstract | 1,2 |
| A | US, A, 4588405 (American Hospital Supply Corporation) 13 May 1986 (13.05.86) see the abstract | 1,2 |
| A | US, A, 4601721 (William Kamerling) 22 July 1986 (22.07.86) see the abstract | 1,2 |

--------------

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 29 May 1987 (29.05.87) | 03 July 1987 (03.07.87) |
| International Searching Authority ISA/SU | Signature of Authorized Officer |

Form PCT/ISA/210 (second sheet) (January 1985)